# FASCICULE DE BREVET EUROPEEN

(11) **EP 3 565 640 B1**
(45) Date de publication et mention de la délivrance du brevet: **02.12.2020**
(21) Numéro de dépôt: 18701408.9
(22) Date de dépôt: 09.01.2018
(51) Int. Cl.: A61Q 7/00, A61K 8/99

(54) **HYDROLYSAT DE PICHIA MINUTA.**
HYDROLYSAT VON PICHIA MINUTA
HYDROLYSATE OF PICHIA MINUTA

(30) Priorité: 09.01.2017 FR 1770024
(43) Date de publication de la demande: 13.11.2019
(73) Titulaire: Societe Industrielle Limousine d'Application Biologique, 19130 Objat (FR)
(72) Inventeur: PAUFIQUE, Jean, 19130 Objat (FR)
(74) Mandataire: Aquinov
(86) Numéro de dépôt international: PCT/EP2018/050462
(87) Numéro de publication internationale: WO 2018/127604

(56) Documents cités:
- WO-A1-2011/103624
- FR-A1- 2 938 768
- FR-A1- 3 016 521

## Description

La présente invention concerne un principe actif cosmétique particulier obtenu par hydrolyse d'une levure et son utilisation pour lutter contre la chute des cheveux et favoriser leur repousse.

Dans une société qui accorde une place prépondérante à l'apparence, la chevelure est un symbole de beauté et de force dont l'état a un impact important sur l'estime de soi et l'image que l'on renvoie. L'accélération de la chute des cheveux, scientifiquement appelée alopécie, est perçue comme un signe de vieillissement anticipé et peut considérablement affecter la qualité de vie des personnes touchées.

C'est pourquoi, de nombreux produits cosmétiques visant à ralentir la chute des cheveux sont proposés, avec une très forte augmentation ces dix dernières années.

L'alopécie est un trouble fréquent dont la forme androgénétique est la plus courante. Elle touche jusqu'à 30% des hommes de moins de 30 ans et plus de 50% des hommes après 50 ans. Ce désordre progressif, qui n'est pas une maladie (Prie B. et al., « Oxidative stress in androgenetic alopecia », Journal of Medicine and Life Vol. 9, Issue 1, January-March 2016, pp. 79-83), est caractérisé par l'apparition de cheveux plus fins, plus courts et non pigmentés formant un duvet. Il résulte de deux principaux facteurs, génétique et hormonal, qui provoquent une hypersensibilité à une hormone : la dihydrotestostérone (DHT). Cette dernière est responsable de l'ensemble des modifications biologiques associées à ce trouble. De plus, certains paramètres environnementaux peuvent également favoriser l'apparition de l'alopécie androgénétique (AGA). C'est le cas de l'utilisation répétée de produits capillaires agressifs (colorants et autres agents chimiques), du tabagisme ou de l'exposition aux UV.

Le maintien d'une chevelure dense est le résultat d'un équilibre entre la perte et la repousse des cheveux. Leur chute est un phénomène naturel puisque chaque jour un individu en perd entre 50 et 125. Grâce à un processus de régénération, leur renouvellement constant est assuré : on parle de cycle de vie du cheveu ou cycle pilaire. Celui-ci se divise en trois phases :
- anagène (croissance) ;
- catagène (dégénérescence) ;
- télogène (latence).

Le cheveu ou follicule pileux est constitué de deux principaux éléments. La papille dermique est l'élément moteur de sa croissance et se situe à la base du follicule. Elle est composée de fibroblastes capables d'émettre des signaux de croissance. Ceux-ci sont ensuite captés par les cellules de la matrice pilaire. Cette structure qui entoure la papille abrite notamment des kératinocytes. En réponse à ces signaux, ils vont proliférer afin de déclencher la formation d'une nouvelle tige pilaire.

La croissance pilaire est un processus énergivore qui nécessite que les cellules disposent de mitochondries fonctionnelles, l'état morphologique de ces organites dynamiques conditionnant la production énergétique.

D'autres facteurs de régulation jouent un rôle majeur dans la régénération du cheveu et présentent un défaut de fonctionnement au cours de l'alopécie androgénétique :
- les molécules « signal », qui permettent aux cellules de communiquer entre elles ; au sein du follicule pileux, elles orchestrent l'action des fibroblastes de la papille et des kératinocytes de la matrice afin d'assurer la croissance pilaire ;
- l'épigénétique.

Actuellement, deux molécules chimiques sont utilisées dans le traitement de l'alopécie androgénétique : le minoxidil et le finastéride. Toutefois, elles sont efficaces dans moins de 50% des cas et présentent des effets secondaires importants (cheveux gras, apparition de pellicules, irritations cutanées, problèmes de libido). Il existe donc un réel besoin de proposer une nouvelle approche permettant de favoriser la croissance des cheveux tout en évitant les désagréments cités précédemment.

Pour y répondre, l'invention propose un principe actif cosmétique naturel, issu d'une levure, capable d'agir sur trois mécanismes de régulation de la repousse du cheveu.

Plusieurs hydrolysats de levures ont déjà été utilisés en cosmétique, tels que des hydrolysats de *Saccharomyces cerevisiae,* de *Pichia anomala* ou de *Torulaspora delbrueckii.* Toutefois, l'invention vise un hydrolysat obtenu à partir d'une levure différente à savoir *Pichia minuta,* qui est éloignée des levures déjà connues et utilisées en cosmétique et qui présente des caractéristiques propres.

En particulier, l'invention vise un principe actif cosmétique constitué par un hydrolysat de *Pichia minuta,* notamment un hydrolysat de *Pichia minuta* comprenant au moins des peptides. Avantageusement, un tel principe actif est capable à la fois de stimuler la dynamique mitochondriale en faveur de la croissance pilaire et de corriger les défauts d'expression touchant les molécules « signal » et l'épigénétique associés à l'alopécie androgénétique. Le principe actif cosmétique selon l'invention permet ainsi de réactiver la papille dermique et de stimuler la croissance du follicule pileux.

L'invention a par conséquent également pour objet l'utilisation cosmétique non thérapeutique d'un tel principe actif, en application sur les cheveux et/ou le cuir chevelu, pour lutter contre la chute des cheveux et activer leur repousse.

L'invention vise aussi les compositions cosmétiques comprenant au moins 0,05% en poids du principe actif selon l'invention, ainsi qu'un procédé cosmétique non thérapeutique pour lutter contre la chute des cheveux et activer leur repousse consistant à appliquer sur les cheveux ou le cuir chevelu de telles compositions.

D'autres caractéristiques et avantages ressortiront de la description en détail de l'invention qui va suivre, en regard des figures annexées sur lesquelles :
- La Figure 1 représente le profil chromatographique (HPLC/RI) des peptides de l'hydrolysat de l'exemple 1,
- La Figure 2 représente l'aminogramme des peptides du principe actif selon l'invention de l'exemple 1,
- La Figure 3 représente le chromatogramme des protéines des trois produits présentés dans l'essai comparatif 1, obtenus selon le même procédé à partir de 3 levures différentes : *Pichia minuta, Pichia heedii* et *Saccharomyces cerevisiae,*
- La Figure 4A représente un milieu de culture gélosé sur lequel a été ensemencé et incubé pendant 48h à 30°C une biomasse de *Pichia minuta,*
- La Figure 4B représente un milieu de culture gélosé sur lequel a été ensemencé et incubé pendant 48h à 30°C un hydrolysat de *Pichia minuta,*
- La Figure 5A représente une photo zoomée d'un cuir chevelu avant application d'une composition selon l'invention,
- La Figure 5B représente une photo zoomée d'un cuir chevelu après 8 mois d'application d'une composition selon l'invention (point II.1)
- La Figure 6A représente une photo d'un cuir chevelu avant application d'une composition selon l'invention,
- La Figure 6B représente une photo d'un cuir chevelu après 8 mois d'application d'une composition selon l'invention (point II.1)

### DEFINITIONS

Par « actif cosmétique » ou « principe actif cosmétique » au sens de l'invention, on entend au moins une molécule, préférentiellement un ensemble de molécules présentant un effet sur les cellules du cheveu et/ou du cuir chevelu, en particulier sur les fibroblastes de la papille dermique et les cellules de la matrice pilaire.

Par « fraction protéique » ou « composés peptidiques » de l'hydrolysat de *Pichia minuta* au sens de l'invention on entend l'ensemble des protéines et peptides présents dans l'hydrolysat de *Pichia minuta.*

Par « hydrolysat de *Pichia minuta* » au sens de l'invention, on entend tout principe actif issu de la levure *Pichia minuta,* obtenu par un procédé comprenant au moins une étape d'hydrolyse enzymatique ou chimique de *Pichia minuta.* Le terme hydrolysat de *Pichia minuta* exclut les molécules produites uniquement par fermentation de *Pichia minuta.*

Par « *Pichia minuta* » au sens de l'invention, on entend toute levure de la famille des *Saccharomycetaceae,* du genre Ogataea et de l'espèce *Pichia minuta.* La levure *Pichia minuta* a été enregistrée dans plusieurs collections de levures, sous les numéros ATCC 26176, CBS 6511, MUCL 27758, MUCL 29976, DSM-70275, NRRL Y-411, NRRL Y-7953, NRRL Y-10948, Y-172, Y-2081, Y-2516,NCYC-499, 3622T, 9442, 3615T, NBRC 1473, NBRC 0975T, NBRC 10402, NBRC 10746. Elle est également connue sous d'autres noms : *Candida methanolovescens, Ogataea minuta, Ogataea nonfermentans. Pichia minuta* peut notamment être isolée à partir de la fleur d'azalée, Rhododendron indicum.

Par « support d'atomisation » au sens de l'invention on entend un adjuvant neutre ajouté dans une solution afin de réaliser une poudre lors du séchage par atomisation.

### DESCRIPTION DETAILLEE DE L'INVENTION

L'invention se rapporte donc à un principe actif cosmétique constitué par un hydrolysat de *Pichia minuta.*

L'hydrolysat de *Pichia minuta* comprend au moins des peptides. Préférentiellement l'hydrolysat de *Pichia minuta* comprend des peptides ayant un poids moléculaire inférieur à 3500 Da, préférentiellement compris entre 243 et 3500 Da. Ces peptides jouent un rôle important dans l'efficacité du principe actif selon l'invention.

De façon préférée, les peptides ayant un poids moléculaire inférieur à 3500 Da représentent au moins 50% en poids de la fraction protéique de l'hydrolysat, encore plus préférentiellement au moins 80%.

Dans l'hydrolysat, la fraction protéique représente au moins 40% en poids de matière sèche de l'hydrolysat, préférentiellement entre 40 et 90%, encore plus préférentiellement entre 50 et 70%.

La teneur en composés peptidiques est préférentiellement déterminée par la méthode de LOWRY (Lowry et al., Protein measurement with the folin reagent, J. Biol. Chem., 193, 265, 1951).

Les acides aminés présents à plus de 5%, constitutifs des peptides de l'hydrolysat (pourcentage par rapport à l'ensemble des acides aminés présents en nombre) sont préférentiellement : alanine, arginine, acide aspartique, acide glutamique, leucine, lysine, serine, thréonine, valine.

L'aminogramme des peptides constituant l'hydrolysat selon l'invention peut être obtenu par chromatographie d'échange d'ions après hydrolyse acide de l'échantillon.

L'hydrolysat comprend également préférentiellement des sucres et/ou des minéraux. Préférentiellement, les sucres représentent au moins 5% en poids de matière sèche de l'hydrolysat, en particulier entre 5 et 25%, encore plus préférentiellement entre 10 et 20 %. La teneur en sucres dans l'hydrolysat peut être déterminée par la méthode de DUBOIS (Dubois M. et al., Analytical chemistry, 28, 3, 350-356, 1956).

Les minéraux présents dans l'hydrolysat sont préférentiellement notamment le calcium, le potassium, le sodium, le chlore, le phosphore. L'analyse des minéraux constituant les cendres de l'hydrolysat a été réalisée par spectrométrie d'émission optique (ICP/OES) et le dosage des ions chlorures par titration au nitrate d'argent.

Préférentiellement, le taux de cendres est compris entre 5 et 35% en poids de matière sèche de l'hydrolysat, encore plus préférentiellement compris entre 20 et 30%.

La teneur en cendres brutes peut être déterminée par la pesée des résidus issus de l'incinération des échantillons de l'hydrolysat à 550°C dans un four à moufle électrique. L'hydrolysat selon l'invention peut être obtenu par une hydrolyse chimique (acide ou basique) ou enzymatique. Préférentiellement, il s'agit d'un hydrolysat enzymatique.

Le principe actif selon l'invention peut se présenter sous forme solide ou sous forme liquide. Lorsqu'il se présente sous forme liquide, le principe actif selon l'invention est préférentiellement l'hydrolysat tel que décrit précédemment. II se présente sous forme d'un liquide limpide, avec une faible odeur et une couleur jaune légèrement orangée.

Lorsqu'il se présente sous forme solide le principe actif selon l'invention est préférentiellement constitué par l'hydrolysat de *Pichia minuta* tel que précédemment décrit et par un support choisi parmi la maltodextrine, la gomme arabique, la lécithine de soja ou l'isomalt. Selon un mode de réalisation, particulièrement adapté, l'hydrolysat représente entre 25 et 75% en poids du principe actif, préférentiellement entre 40 et 60%.

Dans le cas d'une forme solide où le principe actif est associé à un support, les teneurs en protéines, en sucres et en cendres dans le principe actif sont modifiées, le support étant généralement constitué majoritairement de sucres. Dans le cas où le support d'atomisation est la maltodextrine, présent entre 25 et 75%, préférentiellement entre 40 et 60% :
- les peptides représentent au moins 12%, en particulier entre 12 et 53% en poids de matière sèche du principe actif, préférentiellement entre 20 et 42%,
- les sucres représentent entre 32 et 80% en poids de matière sèche du principe actif, préférentiellement entre 46 et 68%,
- les cendres représentent entre 5 et 23% en poids de matière sèche du principe actif, préférentiellement entre 8 et 18%.

L'hydrolysat constituant le principe actif selon l'invention peut être obtenu par tout procédé comprenant au moins une étape d'hydrolyse de *Pichia minuta,* en particulier une étape d'hydrolyse des protéines. Préférentiellement, il est obtenu par un procédé comprenant une étape d'hydrolyse enzymatique, en particulier d'hydrolyse enzymatique des protéines c'est-à-dire une hydrolyse réalisée par voie enzymatique au moyen d'enzymes protéolytiques. Il peut s'agir par exemple de protéases d'origine végétale ou issues de microorganismes.

Préalablement au procédé d'obtention de l'hydrolysat en tant que tel, il convient de produire la biomasse de *Pichia minuta.* Cette étape est réalisée selon le mode de culture des levures dans un milieu adapté à leur développement, de manière classique pour l'homme de métier. Une fois la biomasse obtenue, on réalise une hydrolyse en vue d'obtenir des molécules actives. Selon un mode de réalisation particulièrement adapté, le principe actif est obtenu par la mise en œuvre des étapes suivantes :
- solubilisation de la biomasse de *Pichia minuta* dans l'eau
- hydrolyse des protéines : les conditions d'hydrolyses sont choisies pour hydrolyser à la fois les parois des levures et le milieu intracellulaire des levures, afin de favoriser l'enrichissement en peptides bioactifs ; préférentiellement l'hydrolyse est réalisée de par voie enzymatique au moyen d'enzymes protéolytiques, préférentiellement d'origine végétale ou issues de micro-organismes ;
- inactivation de(s) l'enzyme(s) préférentiellement par traitement thermique : cette inactivation est réalisée selon la préconisation technique du(des) fournisseur(s) de(s) l'enzyme(s) ;
- séparation des phases soluble et insoluble préférentiellement par centrifugation, et récupération de la phase soluble contenant entre autres les peptides et protéines solubles,
- filtration pour éliminer les particules encore en suspension,
- purification par filtration afin d'éliminer, les molécules de haut poids moléculaire (les enzymes et les polymères...), préférentiellement les molécules de poids moléculaire supérieur à 5000Da,
- obtention d'un filtrat, l'hydrolysat de *Pichia minuta,* qui constitue une première forme du principe actif selon l'invention, qui se présente sous forme liquide.

L'hydrolysat obtenu à ce stade peut être encore concentré et/ou purifié afin de sélectionner les fractions de bas poids moléculaires, préférentiellement inférieures à 3 500 kDa, voire éventuellement inférieures à 2000 kDa, par des étapes d'ultrafiltrations successives à travers des filtres de porosité différente, en conservant les filtrats à chaque étape et/ou par une méthode de type chromatographique, afin, par exemple d'enrichir spécifiquement l'hydrolysat en ces molécules.

L'hydrolysat peut ensuite être séché et associé à un support, pour se présenter sous forme solide. Cette phase peut être réalisée par la mise en œuvre des étapes suivantes :
- un support d'atomisation, de préférence la maltodextrine, est ajouté dans l'hydrolysat de *Pichia minuta,* entre 25 et 75% (en masse/volume);
- cette solution est ensuite concentrée sous vide ;
- une débactérisation est réalisée par traitement thermique ;
- l'atomisation permet d'obtenir une poudre.

Cette présentation de l'hydrolysat peptidique correspond à une forme très concentrée en molécules actives, en particulier en peptides.

Les étapes des procédés décrits ci-avant, prises individuellement sont usuelles dans le domaine des extractions d'actifs à partir de matières premières naturelles et l'homme du métier est à même d'en ajuster les paramètres réactionnels sur la base de ses connaissances générales.

Le principe actif selon l'invention est particulièrement efficace pour lutter contre la chute des cheveux et activer leur repousse. En particulier l'hydrolysat de *Pichia minuta* selon l'invention, lorsqu'il est appliqué sur les cheveux et/ou le cuir chevelu :
- favorise la dynamique mitochondriale, élément clé de la croissance pilaire,
- corrige les modifications d'expression des molécules « signal » apparaissant au cours de l'alopécie androgénétique,
- contrôle les acteurs de l'épigénétique au cours de l'alopécie androgénétique, et
- maintient l'activation de la papille dermique et la croissance du follicule pileux au cours de l'alopécie androgénétique.

La croissance du follicule pileux est un processus nécessitant un apport en énergie important. Au sein de la cellule, ce sont les mitochondries qui assurent la production d'énergie. Pour cela, ces organites sont capables d'adapter leur morphologie et *in fine* leur activité : on parle de dynamique mitochondriale. Celle-ci repose sur deux phénomènes : la fission et la fusion. La fission est définie par la séparation des mitochondries qui sont alors plus petites et qui produisent moins d'énergie. A l'inverse, la fusion correspond au regroupement de mitochondries sous la forme d'un réseau. Elle s'appuie notamment sur l'intervention de la mitofusine 1 (MFN1), une protéine membranaire participant à la fusion des membranes externes des mitochondries. Ce processus leur permet d'intensifier leur métabolisme et de produire plus d'énergie sous forme d'ATP.

La dynamique mitochondriale est impliquée dans la biologie du follicule pileux. En particulier, on sait que la morphologie des mitochondries évolue vers une forme fusionnée dans des cellules de la papille dermique. Ce phénomène s'accompagne d'une augmentation de l'activité enzymatique mitochondriale et de la production d'ATP. Ces changements mitochondriaux permettent de fournir l'énergie nécessaire au passage ou au maintien en phase de croissance du cycle pilaire (Mifude et al. « PDGF-AA-induced filamentous mitochondria benefit dermal papilla cells in cellular migration ». International Journal of Cosmetic Science, 37, 266-271 (2015)).

Avantageusement, le principe actif selon l'invention est capable de stimuler la synthèse de mitofusine 1 dans les fibroblastes de la papille dermique et ainsi de promouvoir la croissance du follicule pileux.

Par ailleurs, les molécules « signal » présentes dans le microenvironnement de la papille dermique sont indispensables à la croissance pilaire. Trois d'entre elles se trouvent dérégulées dans l'alopécie androgénétique :
- l'IL-6 (interleukine 6), qui bloque la prolifération des cellules de la matrice et inhibe la croissance de la tige pilaire ;
- DKK1 (Dickkopf 1), un inhibiteur de la voie Wnt/β-caténine, qui provoque l'apoptose des kératinocytes du follicule et ainsi l'entrée en phase catagène (latence) du cycle pilaire ;
- P16, un inhibiteur du cycle cellulaire, qui est responsable de la sénescence prématurée des fibroblastes de la papille dermique.

Dans l'alopécie androgénétique, ces trois molécules « signal » sont surexprimées. En effet, les fibroblastes de la papille dermique issus de zones alopéciques présentent une augmentation de l'expression de P16 (Bahta A.W & al. « Premature Senescence of Balding Dermal Papilla Cells ln Vitro Is Associated with P16INK4a Expression » Journal of Investigative Dermatology, 128, 1088-1094 (2008)), de la sécrétion d'IL-6 (Kwack M.H& al. «Dihydrotestosterone-Inducible Dickkopf 1 from Balding Dermal Papilla Cells Causes Apoptosis in Follicular Keratinocytes» Journal of Investigative Dermatology, 128, 262-269 (2008)) et de l'expression de DKK1 (Fawzi M.M.T & al. «Assessment of tissue levels of dickkopf-1 in androgenetic alopecia and alopecia areata». Journal of Cosmetic Dermatology, 15, 10-15 (2015)). Ces dérégulations sont également observées suite à un traitement par la DHT et peuvent même être accentuées suite à l'activation de la voie de signalisation des androgènes (Yang & al. « Androgen Receptor Accelerates Premature Senescence of Human Dermal Papilla Cells in Association with DNA Damage". Plos One, 8, 1-10 (2013)).

De façon avantageuse, le principe actif selon l'invention est capable de diminuer la sécrétion d'IL-6 et l'expression de DKK1 et de P16 dans les fibroblastes de la papille dermique. Il permet ainsi de corriger l'expression anormale des molécules « signal » impliquées dans l'alopécie androgénétique.

Selon un autre aspect, l'épigénétique joue également un rôle majeur dans la biologie du cheveu. L'épigénétique étudie les mécanismes capables de réguler l'expression des gènes en réponse à certains stress environnementaux et cela, sans modifier la séquence d'ADN.

L'ensemble des acteurs impliqués sont regroupés sous le nom d'épigénome. Parmi eux, les miRNA occupent une place importante. Ces petites molécules d'ARN sont capables d'inactiver les gènes en fonction de l'environnement en modifiant la stabilité de leurs ARN messagers cibles. Il a récemment été démontré que les miRNA régulent différents processus tels que la prolifération et la différenciation des fibroblastes de la papille dermique et des kératinocytes de la matrice pilaire. De plus, ils régulent l'apoptose au cours de la phase catagène (Andl et al. « MicroRNAs (miRNAs) in the control of HF development and cycling: the nextfrontiers in hair research» Experimental dermatology, 24, 821-826 (2015)).

Dans un contexte alopécique, les fibroblastes de la papille dermique présentent un profil d'expression des miRNA dérégulé. Ces modifications incluent notamment la surexpression du miR-3663-3p et de let-7a-3p (Lee et al. « Analysis of the microRNA expression profile of normal human dermal papilla cells treated with 5α-dihydrotestosterone » Molecular Medicine Reports, 12, 1205-1212 (2015)). Parmi les gènes cibles de ces miRNA, certains sont impliqués dans la voie de signalisation Wnt/β-caténine, une voie essentielle à la croissance pilaire. Elle est activée suite à la fixation d'un ligand de la famille Wnt sur son récepteur spécifique et provoque l'accumulation de la β-caténine. *Infine,* cela mène à la prolifération des fibroblastes de la papille dermique et à l'induction de la phase de croissance pilaire (Ouji et al. « Maintenance of Dermal Papilla Cells by Wnt-10b In Vitro » Methods in Molecular Biology, 1516, 269-277 (2016)).

Avantageusement, le principe actif selon l'invention est capable de limiter l'expression des miRNA 3663-3p et let-7a-3p dans les fibroblastes de la papille dermique. Ainsi, il atténue les dérégulations épigénétiques associées à l'alopécie androgénétique.

On sait également que la papille dermique est le centre de contrôle de la croissance pilaire. Les fibroblastes de la papille émettent des signaux à destination des kératinocytes de la matrice pilaire afin de réguler le développement du cheveu. Dans un contexte normal, celui-ci se déroule selon un cycle constitué de trois phases :
- anagène : phase de croissance du cheveu durant 2 à 5 ans ;
- catagène : phase de dégénérescence allant de quelques jours à quelques semaines ;
- télogène : phase de latence durant environ 3 mois.

Dans l'alopécie androgénétique, l'altération de la dynamique du cycle pilaire constitue une dérégulation majeure. Elle se traduit par une augmentation de la proportion de cheveux en phase télogène (latence), dont la durée est maintenue voire allongée. Cela s'accompagne d'un raccourcissement progressif de la phase anagène (croissance) marqué par une baisse d'expression du versican, protéine spécifique de cette étape (Soma et al. « Hair cycle-specific expression of versican in human hair follicles » Journal of Dermatological Science, 39, 147-154 (2005)). L'ensemble de ces dérégulations est à l'origine d'une miniaturisation du follicule pileux à tel point que les cheveux n'atteignent plus la surface de la peau.

Le principe actif selon l'invention est capable de stimuler la synthèse de versican dans des fibroblastes de la papille dermique et permet ainsi de préserver l'activation de la papille dermique. De plus, il améliore la croissance des follicules pileux et permet de conserver une synthèse importante de versican et de Ki-67. Ainsi, le principe actif selon l'invention, permet de réduire le défaut de croissance associé à l'alopécie androgénétique en maintenant les follicules pileux en phase de croissance.

L'invention vise ainsi l'utilisation d'un principe actif selon l'invention, en application sur les cheveux et/ou le cuir chevelu, pour lutter contre la chute des cheveux et activer leur repousse, en particulier chez des personnes présentant une alopécie androgénétique. Il agit sur les anomalies moléculaires associées à l'alopécie androgénétique en corrigeant les dérégulations touchant les molécules « signal » et l'épigénétique et en stimulant la dynamique mitochondriale. Il est ainsi efficace pour normaliser deux paramètres indispensables au développement du cheveu à savoir : l'activation de la papille dermique et la stimulation de la croissance du follicule pileux.

Le principe actif selon l'invention est préférentiellement utilisé dans des compositions, ces compositions comprenant un milieu cosmétiquement acceptable. Il s'agit de compositions dans différentes formes galéniques, adaptées à une application sur le cuir chevelu et les cheveux.

Ces compositions peuvent se présenter notamment sous forme d'émulsions huile-dans-eau, émulsions eau-dans-huile, émulsions multiples (Eau/Huile/Eau ou Huile/Eau/Huile) qui peuvent être éventuellement des microémulsions ou des nanoémulsions, ou sous forme de solutions, suspensions, hydrodispersions, gels aqueux ou poudres. Elles peuvent être plus ou moins fluides et avoir l'aspect de lotion, shampoing, crème ou mousse.

Il peut s'agir de compositions comprenant au moins 0,05% d'un hydrolysat de *Pichia minuta* selon la présente invention, préférentiellement entre 0,5 et 10%.

Ces compositions comprennent, outre l'actif, un milieu physiologiquement acceptable et de préférence cosmétiquement acceptable, c'est-à-dire qui ne provoque pas de sensations d'inconfort inacceptables pour l'utilisateur telles que des rougeurs, tiraillements ou picotements.

Les compositions selon l'invention peuvent contenir comme adjuvant au moins un composé choisi parmi :
- les huiles, qui peuvent être choisies notamment parmi les huiles de silicone, linéaires ou cycliques, volatiles ou non volatiles ;
- les cires, telles que l'ozokérite, la cire de polyéthylène, la cire d'abeille ou la cire de carnauba,
- les élastomères de silicone,
- les tensioactifs, de préférence émulsionnants, qu'ils soient non ioniques, anioniques, cationiques ou amphotères,
- les co-tensioactifs, tels que les alcools gras linéaires,
- les épaississants et/ou gélifiants,
- les humectants, tels que les polyols comme la glycérine,
- les colorants, les conservateurs, les charges,
- les tenseurs,
- les séquestrants,
- les parfums,
- et leurs mélanges, sans que cette liste soit limitative.

Des exemples de tels adjuvants sont cités notamment dans le Dictionnaire CTFA (*International Cosmetic Ingrédient Dictionary and Handbook* publié par le *Personal Care Product Council).* Bien entendu, l'homme du métier veillera à choisir les éventuels composés complémentaires, actifs ou non-actifs, et leur quantité, de telle sorte que les propriétés avantageuses du mélange ne soient pas, ou sensiblement pas, altérées par l'adjonction envisagée.

Ces compositions sont notamment destinées à être utilisées pour un effet anti-chute et repousse des cheveux.

L'invention vise ainsi également un procédé non thérapeutique, à savoir un procédé cosmétique non thérapeutique pour lutter contre la chute des cheveux et activer leur repousse, qui consiste à appliquer sur les cheveux et/ou le cuir chevelu une composition comprenant un principe actif selon l'invention, préférentiellement au moins une fois par jour pendant au moins un mois. De façon préférée, la composition est une composition selon l'invention.

Afin d'illustrer ces effets cosmétiques sur la lutte contre la chute et l'activation de la repousse des cheveux, les exemples suivants avec leurs résultats d'essais sont présentés.

### EXEMPLES

### Exemple 1 : Principe actif selon l'invention sous forme liquide

Le principe actif selon l'exemple 1 est obtenu par la mise en œuvre des étapes suivantes :
- solubilisation de la biomasse de *Pichia minuta* dans l'eau à raison de 50g/l,
- hydrolyse enzymatique avec une enzyme protéolytique d'origine bactérienne,
- inactivation thermique à 80°C de l'activité enzymatique,
- décantation par centrifugation afin de récupérer le surnageant,
- filtration, et collecte du filtrat,
- filtration stérilisante sur filtre 0.22µ.

L'hydrolysat obtenu se présente sous forme d'un liquide limpide, de couleur jaune légèrement orangé, avec une odeur faible.

Il est constitué par :
- 59% de peptides en poids de matière sèche, (déterminée par la méthode de LOWRY),
- 15% de sucres en poids de matière sèche, (déterminée par la méthode de DUBOIS),
- 26% de cendres en poids de matière sèche, (déterminée par la pesée des résidus issus de l'incinération des échantillons de l'hydrolysat à 550°C dans un four à moufle électrique).

Par ailleurs, les peptides ont été caractérisés de façon à déterminer leurs poids moléculaires et quantifier les différentes fractions protéiques par chromatographie FPLC d'exclusion stérique. L'hydrolysat est analysé selon les conditions suivantes :
- Pompe : FPLC AKTA (Pharmacia)
- Colonne : Superdex Peptides TRICORN 10/300 GL (Pharmacia)
- Phase Mobile : tampon phosphate de potassium 20 mM, pH 7,2 avec 0,25 M NaCl.
- Détecteur : UV à 280 nm
- Débit : 0,5 mL/min
- Volume d'injection : 200 µL

Une courbe de calibration des temps de rétention des marqueurs en fonction de leur masse molaire est construite (fit logarithmique). Le Tableau 1 résume les temps de rétention obtenus pour les différents marqueurs.

**Tableau 1. Temps de rétention des différents marqueurs.**

| **Molécules** | **Poids moléculaire (Da)** | **Temps de rétention (min)** |
|---|---|---|
| Cytochrome C | 12 500 | 20,0 |
| Aprotinine | 6 512 | 23,5 |
| Vitamine B12 | 1355 | 33,4 |
| Cytidine | 243 | 41,4 |

Grâce à la courbe de calibration, les temps de rétention suivants ont pu être estimés :
- protéine de 10 000 Da : tr = 21,4 mn
- protéine de 3 500 Da : tr = 25,2 mn
- protéine de 2 000 Da : tr = 30,3 mn

Ensuite, un programme de fractionnement est construit, en fonction des temps de rétention, dans le but de collecter les fractions suivantes (MM = poids moléculaire) :
∘ fraction 1 : 10 000 Da < MM
∘ fraction 2 : 3 500 Da < MM < 10 000 Da
∘ fraction 3 : 2 000 Da < MM < 3 500 Da
∘ fraction 4 : 243 Da < MM < 2 000 Da
∘ fraction 5 : MM < 243 Da

Les différentes fractions collectées de l'hydrolysat sont quantifiées par dosage spectrophotométrique selon la méthode de LOWRY (Lowry et al., Protein measurement with the folin reagent, J. Biol. Chem., 193, 265, 1951).

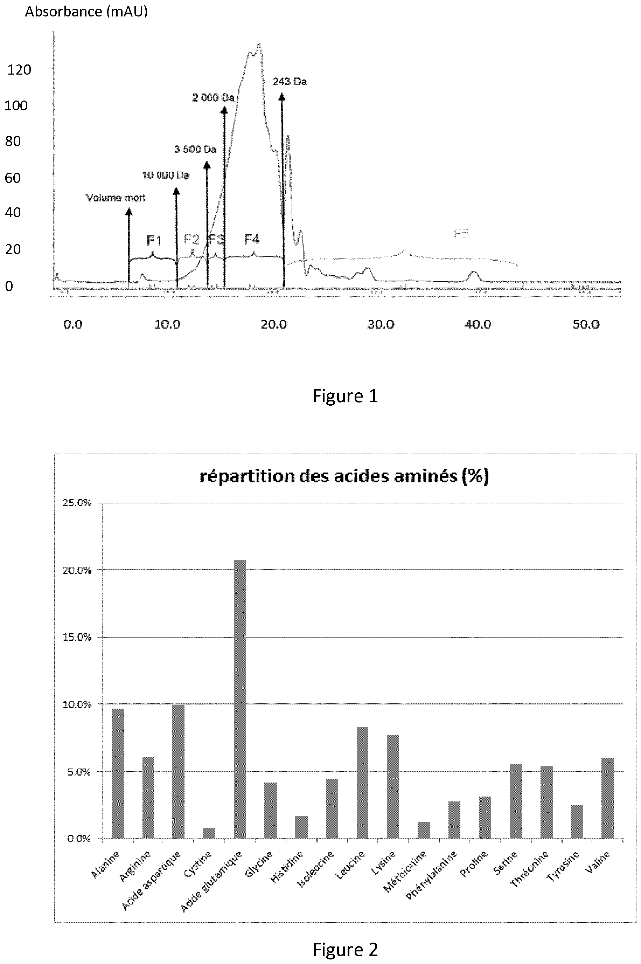

Le profil chromatographique et le fractionnement des peptides de l'hydrolysat sont présentés Figure 1 et la répartition de chaque fraction déterminée par méthode spectrophotométrique est présentée dans le Tableau 2.

**Tableau 2. Répartition et quantification des protéines du principe actif de l'exemple 1**

| **Poids moléculaire (Da)** | **Répartition (%)** |
|---|---|
| Fraction 1 : 10 000 Da < MM | 0,3 |
| Fraction 2 : 3 500 Da < MM < 10 000 Da | 2,9 |
| Fraction 3 : 2 000 Da < MM < 3 500 Da | 10,5 |
| Fraction 4 : 243 Da < MM < 2 000 Da | 83,3 |
| Fraction 5 : MM < 243 Da | 3,0 |

Plus de 93% de peptides du principe actif de l'exemple 1 selon l'invention présentent des masses molaires comprises entre 243 et 3500 Da. La quantité de protéines supérieures à 3500 Da représente moins de 5% des protéines totales.

Un aminogramme a également été réalisé par chromatographie HPLC de l'hydrolyse du produit (acide chlorhydrique concentré pendant 24h) avec une détection à la ninhydrine et les résultats sont présentés dans le Tableau 3 et sur la Figure 2.

**Tableau 3. Aminogramme réalisé sur l'hydrolysat de l'exemple 1**

| | g/100g | Répartition |
|---|---|---|
| Alanine | 2.32 | 9.6% |
| Arginine | 1.46 | 6.1% |
| Acide aspartique | 2.39 | 9.9% |
| Cystine | 0.19 | 0.8% |
| Acide glutamique | 4.99 | 20.9% |
| Glycine | 1.01 | 4.2% |
| Histidine | 0.40 | 1.7% |
| Isoleucine | 1.07 | 4.5% |
| Leucine | 1.99 | 8.3% |
| Lysine | 1.86 | 7.7% |
| Méthionine | 0.29 | 1.2% |
| Phénylalanine | 0.66 | 2.7% |
| Proline | 0.75 | 3.1% |
| Serine | 1.33 | 5.5% |
| Thréonine | 1.30 | 5.4% |
| Tyrosine | 0.60 | 2.5% |
| Valine | 1.45 | 6.0% |
| Total | 24.06 | 100.0% |

Par ailleurs, la répartition des cendres a été déterminée après incinération à 550°C. L'analyse des minéraux a été réalisée par spectrométrie d'émission optique (ICP/OES) et le dosage des ions chlorures par titration au nitrate d'argent.

Elle est présentée dans le Tableau 4 ci-dessous :

**Tableau 4. Répartition des cendres de l'hydrolysat de l'exemple 1**

| | **Répartition des cendres (%)** |
|---|---|
| Calcium | 4 |
| Potassium | 18 |
| Magnésium | 1 |
| Sodium | 14 |
| Chlore | 43 |
| Phosphore | 12 |
| Soufre | 7 |
| Non identifié | 1 |

### Exemple 2 : Principe actif selon l'invention sous forme solide (hydrolysat de l'exemple 1 + maltodextrine)

Le principe actif de l'exemple 1 est associé à la Maltodextrine pour constituer le principe actif de l'exemple 2.

Il est obtenu par la mise en œuvre des étapes suivantes :
- introduction de 50% de maltodextrine (masse) dans l'hydrolysat de l'exemple 1,
- concentration jusqu'à un facteur de concentration de 5,
- débactérisation 5min à 95°C,
- atomisation.

Le principe actif se présente sous forme de poudre présentant une granulométrie inférieure à 500µm, une odeur faible, une couleur jaune légèrement orangé.

Il est constitué par (détermination selon les mêmes méthodes que pour l'hydrolysat à l'exemple 1) :
- 28% de peptides en poids de matière sèche,
- 58% de sucres en poids de matière sèche,
- 14% de cendres en poids de matière sèche.

La maltodextrine n'étant constituée que de sucres non actifs, la composition en peptides, l'aminogramme et la composition en minéraux du principe actif sont identiques à ceux de l'exemple 1.

### Exemple 3 : Sérum repousse des cheveux

Un exemple de composition selon l'invention sous forme de sérum est constitué par :

| | | | |
|---|---|---|---|
| A. | Eau | | qsp 100% |
| | Conservateur | | 0,7% |
| | Propylène glycol | | 30% |
| | | | |
| B. | Simulgel FL10 | (Seppic) | 0,5% |
| | DC200 | (Dow Corning) | 8% |
| | | | |
| C. | Acide lactique | | qsp pH 5 |
| | | | |
| D. | **Principe actif** | **EXEMPLE 2** | 0,3% |

Le pH de la composition est de 5. Elle se présente sous forme d'un liquide opalescent, sans odeur.

Le sérum de l'exemple 3 présente les caractéristiques suivantes : lotion sprayable, application fraîche et soyeuse, effet glissant, pénétration immédiate, fini doux et sec, effet lissant sur les cheveux.

Il peut être obtenu par la mise en œuvre des étapes suivantes :
- Mélanger A et chauffer au bain marie à 50°C, en veillant à bien disperser le conservateur,
- Laisser refroidir sous agitation,
- Ajouter B, sous agitation et ajuster le pH avec C,
- Ajouter D sous agitation à 1500 tr/min et vérifier la complète homogénéisation de la formule.

### Exemple 4 : Masque repousse des cheveux

Un exemple de composition selon l'invention sous forme de masque pour les cheveux, est constitué par :

| | | | |
|---|---|---|---|
| A. | Eau | | qsp 100% |
| | Conservateur | | 0,7% |
| | Propylène glycol | | 20% |
| | Glucose acétate | | 6% |
| B. | **Principe actif** | **EXEMPLE 2** | **0,3%** |
| C. | Simulquat HC305 | (Seppic) | 6% |

Le pH de la composition est de 4,5.

Elle se présente sous forme d'un gel blanc épais et compact.

Le masque de l'exemple 3 présente les caractéristiques suivantes : préhension ferme, application facile, effet filmogène, rinçage aisé, fini doux et sec.

Il peut être obtenu par la mise en œuvre des étapes suivantes :
- Mélanger A et chauffer au bain marie à 50°C, en veillant à bien disperser le conservateur,
- Ajouter B sous agitation et veiller à la bonne homogénéisation,
- Ajouter C sous forte agitation (3000 tr/min) et laisser sous agitation jusqu'à la complète homogénéisation du gel.

### Exemple 5 : Soin repousse du cheveu

Un exemple de composition selon l'invention sous forme de soin pour les cheveux, est constitué par :

| | | | |
|---|---|---|---|
| | Eau | - | qsp |
| A. | | | 100% |
| | Conservateur | - | 0,7% |
| | Dub Diol | (Stéarinerie DUBOIS) | 20% |
| | | | |
| B. | Satiaxane CX930 | (Cargill) | 0,1% |
| | Satialgine US551 | (Cargill) | 3% |
| | | | |
| C. | **Principe actif** | **EXEMPLE 1** | **0,7%** |
| | | | |
| D. | GENUGEL carrageenan CG-130 | (CP Kelco) | 2,5% |
| | Lauroyl Lysine | (Laboratoire HYTECH) | 2% |
| D-panthénol | | (XINFU) | 1% |
| Phytosqualane | | (Sophim) | 1% |

Le pH de la composition est de 6,1.

Elle se présente sous forme d'un gel écru épais et fluide, sans odeur.

Le soin de l'exemple 5 présente les caractéristiques suivantes : Préhension souple, application douce et bien homogène sur la peau et le cheveu, effet frais et hydratant, rinçage aisé, fini doux et lissant.

Il peut être obtenu par la mise en œuvre des étapes suivantes :
- Mélanger A et chauffer au bain marie à 50°C, en veillant à bien disperser le conservateur,
- Ajouter B, sous agitation et veiller à la bonne homogénéisation des gels,
- Ajouter C, et vérifier la bonne dispersion de l'actif,
- A froid, ajouter D, dans l'ordre indiqué et laisser sous agitation (2500 tr/min), jusqu'à totale homogénéisation.

### Exemple 6 : Base lavante traitante

Un exemple de composition selon l'invention sous forme de soin pour les cheveux, est constitué par :

| | | | |
|---|---|---|---|
| A. | Oramix LS30 | (Seppic) | 25% |
| | Betadet S20 | (Kao Corporation) | 25% |
| | Oramix NS20 | (Seppic) | 20% |
| | Somepon T25 | (Seppic) | 4% |
| | | | |
| B. | Conservateur | - | 1% |
| | Carbopol Ultrez10 | (Novéon) | 0,1% |
| | | | |
| C. | **PRINCIPE ACTIF** | **EXEMPLE 1** | **0,7%** |
| | | | |
| D. | NaOH | - | Qsp pH4,8 |
| | | | |
| E. | Dub Grenn OD4 | (Stéarinerie DUBOIS) | 15% |
| Baobab Oil | (Sophim) | | 5% |
| Biophytosebum | (Sophim) | | 5% |

Le pH de la composition est de 5.

Elle se présente sous forme d'une base gélifiée liquide, légèrement écrue, sans odeur.

La base lavante de l'exemple 6 présente les caractéristiques suivantes : écoulement fluide, touché doux, pouvoir moussant extrêmement fort, bonne dispersion à l'eau avec création d'une mousse douce, blanche et compacte, rinçage facile, fini doux très légèrement filmogène, effet hydratant.

Elle peut être obtenue par la mise en œuvre des étapes suivantes :
- Mélanger A sans agiter pour éviter de former une mousse,
- Ajouter B, puis C et agiter délicatement en veillant à la bonne dispersion des poudres,
- Ajuster le pH avec D et sous agitation plus forte (1000 tr/min), ajouter la phase grasse et laisser sous agitation jusqu'à complète homogénéisation du gel.

### Exemple 7 : Lotion traitante

Un exemple de composition selon l'invention sous forme de lotion pour les cheveux, est constitué par :

| | | |
|---|---|---|
| A. | Eau | qsp 100% |
| | Conservateur | 0,7% |
| | Propylène glycol | 8% |
| | **Principe actif EXEMPLE 2** | **0,3%** |
| | | |
| B. | Glycerol | 2,0% |
| | | |
| C. | Alcohol | 10,0% |

Elle se présente sous la forme d'une lotion éthanolique, liquide transparente.

Elle peut être obtenue par la mise en œuvre des étapes suivantes :
- Mélanger A sans agiter pour éviter de former une mousse,
- Ajouter B, puis C, et agiter délicatement en veillant à une bonne dispersion.

### EVALUATION DE L'EFFICACITE COSMETIQUE SELON L'INVENTION

### I. TESTS IN VITRO

### ∘ 1. Effet d'un hydrolysat de Pichia minuta selon l'invention sur la dynamique mitochondriale nécessaire à la croissance du follicule pileux

L'objectif de cette étude est d'évaluer l'effet d'un principe actif selon l'invention sur la dynamique mitochondriale, un élément nécessaire à la croissance du follicule pileux.

Pour cela, la synthèse de mitofusine 1 a été évaluée. La mitofusine 1 est un acteur majeur de la fusion mitochondriale, dans des fibroblastes humains de la papille dermique. Lorsque la croissance pilaire est favorisée, la dynamique des mitochondries est modifiée. Cela se traduit par une augmentation de la proportion de grandes mitochondries fusionnées par rapport aux petites mitochondries fissionnées dans des fibroblastes de la papille dermique. Cet état est favorable à la production d'énergie.

L'étude a été réalisée par Western blot selon le protocole opératoire décrit en suivant.

A J0, les fibroblastes humains de la papille dermique sont ensemencés dans du milieu de culture et incubés à 37°C.

A J3, les cellules sont traitées : les fibroblastes sont traités avec du milieu contenant ou non le principe actif à 0,02% et 0,04% (V/V final).

A J5, les extraits cellulaires sont récupérés puis stockés à -80°C en attente du dosage par Western blot selon les caractéristiques suivantes :
- une électrophorèse est réalisée sur gel précoulé et les protéines sont ensuite transférées sur membrane PVDF,
- un immunomarquage est réalisé à l'aide d'un anticorps primaire anti-mitofuscine 1,
- les bandes sont visualisées, semi-quantifiées par densitométrie avec une caméra CCD, puis analysées à l'aide d'un logiciel.

Les résultats obtenus sont présentés dans le Tableau 5 :

**Tableau 5. Effet d'un hydrolysat de Pichia minuta sur la synthèse de mitofusine 1 par des fibroblastes de la papille dermique**

| | **Taux de mitofusine 1 (UA)** | **Efficacité / Témoin (%)** |
|---|---|---|
| Témoin | 0,929 | |
| Principe actif - Exemple 2 à 0,02% | 1,180 | 27 |
| Principe actif -Exemple 2 à 0,04% | 1,412 | 52 |

Ces résultats montrent que testé à 0,04%, un hydrolysat de *Pichia minuta* permet d'augmenter significativement la synthèse de mitofusine 1 de 52% dans des fibroblastes humains de la papille dermique. Ainsi, il agit favorablement sur la dynamique mitochondriale, élément clé pour la production de l'énergie nécessaire à la croissance du follicule pileux.

### ∘ 2. Effet d'un hydrolysat de Pichia minuta selon l'invention sur la régulation de molécules « signal » décrites dans l'alopécie androgénétique

L'objectif de cette étude est d'évaluer l'effet d'un principe actif selon l'invention sur la régulation de molécules « signal » décrites dans l'alopécie androgénétique.

Pour cela, l'étude a consisté à évaluer sur des fibroblastes de la papille dermique traités avec une solution de DHT mimant l'alopécie :
- l'IL-6 (interleukine 6), cytokine sécrétée par les fibroblastes humains de la papille dermique qui inhibe la croissance pilaire ;
- DKK1 (Dickkopf 1), inhibiteur de la voie Wnt/β-caténine, acteur majeur de l'alopécie ;
- P16, protéine du cycle cellulaire, associée à la sénescence prématurée des fibroblastes de la papille dermique de zone alopécique.

Cette étude a été réalisée par dosage ELISA et PCR quantitative, selon le protocole opératoire décrit en suivant.

A J0, les fibroblastes humains de la papille dermique sont ensemencés dans du milieu de culture et incubés à 37°C.

A J3, les fibroblastes sont traités avec une solution de DHT contenant ou non le principe actif à 0,02% et 0,04% (V/V final).

A J5, à la fin de l'incubation, les surnageants cellulaires sont récupérés. Le dosage d'IL-6 est réalisé à l'aide d'un kit de dosage ELISA. Les ARN ont été reverses-transcrits et les ADN complémentaires obtenus ont été analysés par la technique de PCR quantitative.

L'expression des ARNm DKK1 et P16 a également été étudiée. En parallèle, les ARNm de la Ribosomal Protein S27 (RPS27), de l'Hypoxanthine Phosphoribosyltransferase 1 (HPRT1), de la bêta-Glucuronidase (GUSB) ont été analysés comme témoins internes de référence pour la normalisation.

La quantification de l'incorporation de fluorescence (SYBR Green) est mesurée en continu à l'aide d'un thermocycleur. L'analyse des Ct (quantification relative) est réalisée à l'aide d'un logiciel.

Les résultats sont présentés dans les Tableaux 6 et 7.

**Tableau 6. Effet d'un hydrolysat de Pichia minuta sur la libération d'interleukine 6 par des fibroblastes de la papille dermique dans un modèle normal ou alopécique.**

| | Taux d'IL-6 (fg/µg de protéines) | Capacité à limiter la sécrétion d'IL-6 (%) |
|---|---|---|
| Modèle normal | | |
| Témoin | 1284 | |
| Principe actif - Exemple 2 0,04% | 1182 | |

| Modèle alopécique | | |
|---|---|---|
| Témoin | 1736 | |
| Principe actif-Exemple 2 0,02% | 1431 | 67 |
| Principe actif-Exempte 2 0,04% | 1299 | 97 |

**Tableau 7. Effet d'un hydrolysat de Pichia minuta sur l'expression de DKK1 et P16 par des fibroblastes de la papille dermique dans un modèle normal ou alopécique.**

| | DKK1 (%) | Capacité à limiter l'expression de DKK1 (%) | P16 (%) | Capacité à Iimiter10 l'expression de P16 (%) |
|---|---|---|---|---|
| Modèle normal | | | | |
| Témoin | 100 | | 100 | |
| Principe actif - Exemple 2 0,04% | 92 | | 105 | |

| Modèle alopécique | | | | |
|---|---|---|---|---|
| Témoin | 212 | | 195 | |
| Principe actif-Exemple 2 0,02% | 132 | 71 | 139 | 59 |
| Principe actif - Exemple 2 0,04% | 110 | 91 | 128 | 71 |

On constate que la DHT augmente significativement la sécrétion d'IL-6 et l'expression de DKK1 et P16 par les fibroblastes humains de la papille dermique, mimant ainsi la modification d'expression de molécules « signal » décrites dans l'alopécie androgénétique.

Testé à 0,04%, le principe actif selon l'invention permet de limiter significativement la sécrétion d'IL-6 de 97% et les expressions de DKK1 et de P16 de respectivement 91% et 71%. Ainsi, il corrige la production anormale de molécules « signal » intervenant dans l'alopécie androgénétique.

### ∘ 3. Effet d'un hydrolysat de Pichia minuta selon l'invention sur les modifications épigénétiques associées à l'alopécie androgénétique

L'objectif de cette étude est d'évaluer l'effet d'un principe actif selon l'invention sur les modifications épigénétiques associées à l'alopécie androgénétique.

Pour cela, l'expression de miRNA dans un modèle de fibroblastes humains de papille dermique, traités avec une solution de dihydrotestostérone (DHT) (afin de mimer l'alopécie) a été évalué. Cette étude est focalisée sur deux miRNA impliqués dans la croissance du cheveu : 3663-3p et let-7a-3p dont les expressions sont induites par la DHT. Parmi les voies biologiques ciblées par ces miRNA, on trouve notamment la voie de signalisation Wnt/βcaténine essentielle pour la croissance des cheveux.

L'étude a été réalisée par PCR quantitative, selon le protocole opératoire suivant.

A J0, les fibroblastes humains de la papille dermique sont ensemencés dans du milieu de culture et incubés à 37°C dans une atmosphère contenant 5% de CO₂.

A J3, les fibroblastes sont traités avec une solution de DHT contenant ou non le principe actif selon l'invention à 0,02% et 0,04% (V/V final).

AJ4, les ARN ont été reverses-transcrits et les ADN complémentaires obtenus ont été analysés par la technique de PCR quantitative.

L'expression des microRNA : 3663-3p et let-7a-3p a été étudiée.

En parallèle, small nucleolar RNA C/D box 68 (SNORD68) a été analysé comme témoin interne de référence pour la normalisation,

La quantification de l'incorporation de fluorescence est mesurée en continu à l'aide d'un thermocycleur. L'analyse des Ct (quantification relative) est réalisée à l'aide d'un logiciel.

Les résultats sont donnés dans le Tableau 8.

**Tableau 8. Effet d'un hydrolysat de Pichia minuta sur l'expression des miRNA 3663-3p et let-7a-3p par des fibroblastes de la papille dermique dans un modèle normal ou alopécique.**

| | miRNA 3663-3p (%) | Capacité à limiter l'expression de miRNA 3663-3p (%) | miRNA let-7a-3p (%) | Capacité à limiter l'expression de miRNA let-7a-3p (%) |
|---|---|---|---|---|
| Modèle normal | | | | |
| Témoin | 100 | | 100 | |
| Principe actif-Exemple 2 0,04% | 86 | | 120 | |

| Modèle alopécique | | | | |
|---|---|---|---|---|
| Témoin | 172 | | 172 | |
| Principe actif-Exemple 2 0,02% | 143 | 40 | 125 | 65 |
| Principe actif - Exemple 2 0,04% | 136 | 50 | 108 | 89 |
| Principe actif-Exemple 1 0.04% | 133 | 54 | | |

La DHT induit l'expression des miRNA 3663-3p et let-7a-3p dans des fibroblastes humains de la papille dermique, mimant ainsi les modifications épigénétiques associées à l'alopécie androgénétique.

On constate que, testé à 0,04%, un hydrolysat de *Pichia minuta* permet de réduire significativement l'expression des miRNA 3663-3p et let-7a-3p de respectivement 50% et 89%. Ainsi, un principe actif selon l'invention permet de limiter les modifications épigénétiques associées à l'alopécie androgénétique.

### ∘ 4. Effet d'un hydrolysat de Pichia minuta selon l'invention sur l'activité de la papille dermique dans l'alopécie androgénétique

L'objectif de cette étude est d'évaluer l'effet d'un principe actif selon l'invention sur l'activation de la papille dermique.

Pour cela, la synthèse de versican a été mesurée. Il s'agit d'un protéoglycane spécifique de la phase de croissance. L'évaluation a été réalisée dans un modèle 3D de fibroblastes sous forme de sphéroïdes, traités avec une solution de DHT. Ce modèle mime ainsi l'inactivation de la papille dermique, facteur contribuant au défaut de croissance associé à l'alopécie androgénétique.

L'étude a été réalisée par marquage immunohistologique selon le protocole opératoire suivant.

A J0, les fibroblastes humains de la papille dermique sont ensemencés dans du milieu de culture et incubés à 37°C.

A J1, les sphéroïdes formées sont traitées avec une solution de DHT contenant ou non le principe actif selon l'invention à 0,04% (V/V final).

A J4, les sphéroïdes sont récupérées et fixées.

Un marquage immunohistologique de versican est réalisé. La visualisation est effectuée sur un microscope confocal couplé à un système d'analyse d'images.

La synthèse de versican est proportionnelle à l'intensité de la fluorescence (couleur verte) présente sur les sphéroïdes.

Les noyaux des cellules apparaissent colorés en bleu.

Les résultats étant qualitatifs, nous avons défini 3 niveaux de synthèse du versican :
- Synthèse faible +
- Synthèse moyenne ++
- Synthèse forte +++

Les résultats sont donnés sur le Tableau 9.

**Tableau 9. Effet d'un hydrolysat de Pichia minuta sur la synthèse de versican dans des sphéroïdes de fibroblastes de papille dermique dans un modèle alopécique.**

| | Synthèse de versican |
|---|---|
| Modèle normal | |
| Témoin | +++ |

| Modèle alopécique | |
|---|---|
| Témoin | + |
| Principe actif-exemple 2 0,04% | ++ |

La DHT induit une diminution de la synthèse de versican, dans un modèle de fibroblastes humains de papille dermique sous forme de sphéroïdes, mimant une inactivation de la papille dermique induite au cours de l'alopécie androgénétique.

On constate que testé à 0,04%, le principe actif selon l'invention stimule la synthèse de versican dans ce modèle. Ainsi, il permet de maintenir l'activité de la papille dermique et sa capacité à induire la croissance du follicule pileux.

### ∘ 5. Effet d'un hydrolysat de Pichia minuta selon l'invention sur la croissance du follicule pileux dans l'alopécie androgénétique

L'objectif de cette étude est d'évaluer la capacité d'un principe actif selon l'invention, à maintenir le follicule pileux dans une phase de croissance (phase anagène).

Pour cela, nous avons réalisé une étude sur le modèle de culture de follicules pileux *ex vivo* développé par Philpott *et al.* traités avec une solution de DHT afin de mimer la réduction de croissance associée à l'alopécie androgénétique. Cette capacité a été évaluée en mesurant :
- l'élongation du follicule pileux ;
- la synthèse de versican et de Ki-67, deux protéines clés de la croissance pilaire.

L'évaluation de l'élongation est réalisée par mesure de la taille des follicules pileux après photographies. Les synthèses de versican et de Ki-67 sont étudiées par marquages immunohistologiques. Le protocole opératoire est décrit en suivant.

A J0, les follicules pileux sont obtenus à partir de fragments de cuir chevelu et incubés dans du milieu de culture à 37°C.

A J1, les follicules sont photographiés et mesurés sur microscope couplé à un système d'analyse d'images.

Les follicules sélectionnés (en phase anagène) sont ensuite traités avec une solution de DHT contenant ou non le principe actif selon l'invention à 0,02% et 0,04% (V/V final).

A J5, deux études sont réalisées : l'étude de l'élongation du follicule pileux et l'étude de la synthèse de versican et de Ki-67.

### - Etude de l'élongation du follicule pileux

Les follicules sont photographiés et mesurés sur microscope couplé à un système d'analyse d'images. L'élongation de chaque follicule est égale à sa croissance entre J0-J5.

Les follicules sont traités avec une solution de DHT contenant ou non le principe actif à 0,02% et 0,04% (V/V final).

### - Etude de la synthèse de versican et de Ki-67

Les follicules sont récupérés et inclus en Tissue-Tek® puis congelés. Des coupes (4 µm) sont ensuite réalisées à l'aide d'un cryostat. Un marquage immunohistologique de versican et Ki-67 est réalisé. La visualisation est effectuée sur un microscope confocal couplé à un système d'analyse d'images.

La synthèse de versican est proportionnelle à l'intensité de la fluorescence (couleur verte) présente sur les coupes de follicules pileux.

La synthèse de Ki67 est proportionnelle à l'intensité de la fluorescence (couleur jaune) présente sur les coupes de follicules pileux.

A J7, les follicules sont de nouveau photographiés et mesurés. L'élongation de chaque follicule est égale à sa croissance entre J0-J7.

Les résultats de l'effet sur l'élongation du follicule pileux sont donnés dans le tableau 10.

**Tableau 10. Effet d'un hydrolysat de Pichia minuta sur la croissance des follicules pileux ex vivo dans un modèle alopécique.**

| | | |
|---|---|---|
| | Elongation (µm) | Capacité à restaurer l'élongation (%) |

| **J0-J5** | | |
|---|---|---|
| Modèle normal | | |
| Témoin | 1201 | |

| Modèle alopécique | | |
|---|---|---|
| Témoin | 743 | |
| Principe actif - Exemple 2 0,02% | 822 | +11 |
| Principe actif - Exemple 2 0,04% | 887 | +31 |
| | | |

| **J0-J7** | | |
|---|---|---|
| Modèle normal | | |
| Témoin | 1617 | |

| Modèle alopécique | | |
|---|---|---|
| Témoin | 961 | |
| Principe actif-Exemple 2 0,02% | 1038 | +12 |
| Principe actif - Exemple 2 0,04% | 1206 | +37 |

On constate que la DHT réduit significativement la croissance des follicules pileux à J5 et J7, de respectivement -31% et -37%. De ce fait, la DHT mime le défaut de croissance pilaire associé à l'alopécie androgénétique.

Testé à 0,04%, un principe actif selon l'invention permet d'augmenter significativement la croissance des follicules pileux à J5 et J7, de respectivement +31% et +37%.

Les résultats de l'effet sur la matrice pilaire du follicule pileux sont donnés sur le Tableau 11.

Les résultats étant qualitatifs, 3 niveaux de synthèse du versican et du Ki-67 ont été définis :
- Synthèse faible +
- Synthèse moyenne ++
- Synthèse forte +++

**Tableau 11. Effet d'un hydrolysat de Pichia minuta sur les synthèses de versican et Ki-67 par des follicules pileux ex vivo dans un modèle alopécique.**

| | Synthèse de versican et Ki-67 |
|---|---|
| Modèle normal | |
| Témoin | +++ |

| Modèle alopécique | |
|---|---|
| Témoin | + |
| Principe actif - Exemple 2 0,04% | ++ |

On constate que la DHT réduit significativement les synthèses de versican et de Ki-67. De ce fait, la DHT mime les altérations métaboliques associées à l'alopécie androgénétique.

Testé à 0,04%, un principe actif selon l'invention permet de conserver une synthèse de versican importante, et le pouvoir prolifératif des kératinocytes.

Ainsi, l'utilisation selon l'invention permet de limiter la réduction de croissance associée à l'alopécie androgénétique et maintient les follicules pileux dans une phase anagène

### II. TESTS IN VIVO

Les volontaires participant aux tests in vivo ont été sélectionnés selon plusieurs critères. Ce sont des hommes de moins de 55 ans, présentant une alopécie légère à modérée et ayant un minimum de 20% de cheveux en phase télogène.

### ∘ 1. Effet d'un hydrolysat de Pichia minuta selon l'invention sur la croissance capillaire

L'objectif de cette étude a été d'évaluer, in *vivo,* l'effet anti-chute d'un principe actif selon l'invention (exemple 2) formulé à 0,3% dans une lotion (exemple 7), chez des volontaires présentant une alopécie légère à modérée.

Cette étude a été réalisée sur 22 volontaires d'âge compris entre 31 et 55 ans (âge moyen 43 ± 8 ans), ayant appliqué matin et soir 1 mL de la lotion selon l'invention pendant 8 mois. L'effet anti-chute a été étudié par la méthode du phototrichogramme à partir de mesures effectuées à T0, 3, 6 et 8 mois après le début du traitement. Les paramètres suivants ont été analysés :
- densité capillaire,
- nombre de cheveux en phase anagène : indicateur de la croissance des cheveux ;
- nombre de cheveux en phase télogène : indicateur de la chute des cheveux ;
- ratio Anagène / Télogène : coefficient de croissance du cheveu.

Un résumé des résultats sur la densité capillaire est présenté dans le Tableau 12.

**Tableau 12. Effet d'un hydrolysat de Pichia minuta formulé à 0,3% sur la densité capillaire après 3, 6 et 8 mois d'applications biquotidiennes.**

| | Variation / J0 (%) |
|---|---|
| 3 mois | +21,3 |
| 6 mois | +22,1 |
| 8 mois | +24,9 |

On constate qu'un principe actif selon l'invention, formulé à 0,3% augmente la densité capillaire dès 3 mois d'application (+21,3%). Cet effet se prolonge et s'intensifie après 6 et 8 mois de traitement (respectivement +22,1 et +24,9%).

Un résumé des résultats sur la croissance capillaire est présenté dans le Tableau 13.

**Tableau 13. Effet d'un hydrolysat de Pichia minuta formulé à 0,3% sur la croissance capillaire après 3, 6 et 8 mois d'applications biquotidiennes.**

| | Variation / J0 (%) | |
|---|---|---|
| | Cheveux en phase de croissance (phase anagène) (%) | Amélioration du ratio Anagène / Télogène (%) |
| 3 mois | +24,7 | +73,1 |
| 6 mois | +38,7 | +85,7 |
| 8 mois | +49,1 | +112,7 |

On constate que le principe actif selon l'invention montre une action sur la croissance capillaire, dès 3 mois d'application, qui s'intensifie au cours du traitement.

Cela se traduit par une augmentation statistiquement significative de la proportion de cheveux en phase anagène. De plus, on note une augmentation significative du ratio Anagène / Télogène.

Un résumé des résultats sur la diminution du nombre de cheveux en phase télogène capillaire est présenté dans le Tableau 14. Ce paramètre, caractéristique d'une réduction de la chute de cheveux, a été évalué par phototrichogramme.

**Tableau 13. Effet d'un hydrolysat de Pichia minuta formulé à 0,3% sur la diminution des cheveux en phase télogène après 3, 6 et 8 mois d'applications biquotidiennes.**

| | Variation / J0 (%) |
|---|---|
| 3 mois | -14,0 |
| 6 mois | -21,9 |
| 8 mois | -26,1 |

Dans les conditions de cette étude, le principe actif selon l'invention formulé à 0,3% dans une lotion, diminue significativement la proportion de cheveux en phase télogène dès 3 mois d'application (-14,0%,). Cet effet s'intensifie après 6 et 8 mois de traitement (respectivement -21,9% et-26,1%).

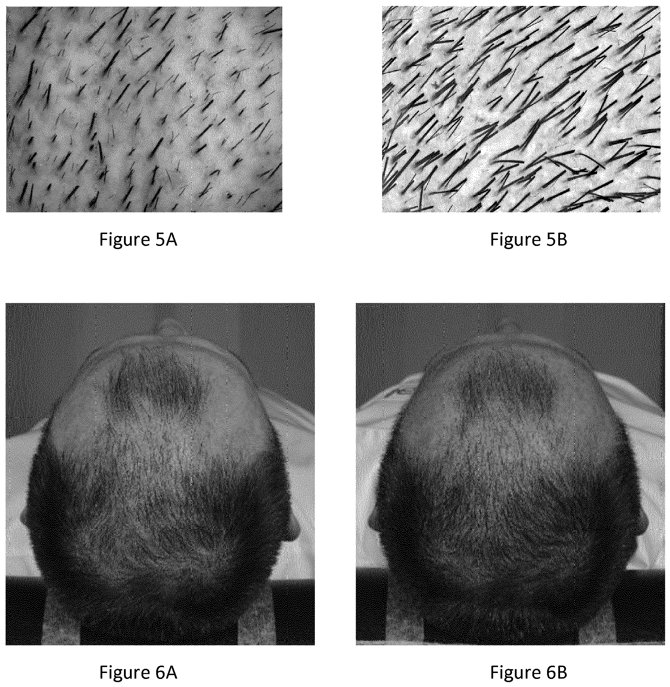

Ces différents résultats sont illustrés sur les Figures 5A / 6A (à J0 avant application) et 5B / 6B (après 8 mois d'application). Le gain total de cheveux sur le cuir chevelu entre J0 et J8mois pour cette illustration est de 74 160 cheveux.

En stimulant la croissance des cheveux (phase anagène) et en diminuant la proportion de cheveux en phase télogène, l'utilisation d'un principe actif selon l'invention permet de renforcer et redensifier la chevelure.

### ∘ 2. Effet d'un hydrolysat de Pichia minuta selon l'invention sur la chute des cheveux

L'objectif de cette étude a été d'évaluer, in *vivo,* la capacité d'un principe actif selon l'invention (exemple 2) formulé à 0,3% dans une lotion (exemple 7), à freiner la chute des cheveux de volontaires présentant une alopécie légère à modérée.

Cette étude a été réalisée sur 22 volontaires d'âge compris entre 31 et 55 ans (âge moyen 43 ± 8 ans), ayant appliqué matin et soir 1 mL de la lotion selon l'invention.

L'importance de la chute des cheveux a été étudiée par la méthode du wash test effectuée avant ainsi que 3, 6 et 8 mois après le début du traitement.

Un résumé des résultats correspondant à l'effet d'un principe actif selon l'invention formulé à 0,3% sur la chute des cheveux évaluée par comptage après wash test est présenté dans le Tableau 14.

**Tableau 14. Effet d'un hydrolysat de Pichia minuta formulé à 0,3% dans une lotion sur la chute des cheveux après 3, 6 et 8 mois d'applications biquotidiennes.**

| | Variation / J0 (%) |
|---|---|
| 3 mois | -21,2% |
| 6 mois | -30,9% |
| 8 mois | -34,0% |

Dans les conditions de cette étude, dès 3 mois d'applications biquotidiennes, la composition selon l'invention freine la chute de cheveux en réduisant de 21,2% le nombre de cheveux récupérés à la suite d'un shampooing.

Cet effet se prolonge et s'intensifie après 6 mois de traitement pour atteindre une diminution moyenne de la perte de cheveux de 30,9%, la diminution maximale étant de 81%. Plus de 8 volontaires sur 10 ont présenté cet effet.

Enfin, après 8 mois de traitement, l'invention stabilise la chute de cheveux en limitant de 34,0% le nombre de cheveux perdus.

### ESSAIS COMPARATIFS: COMPARAISON DU PRINCIPE ACTIF SELON L'INVENTION AVEC D'AUTRES HYDROLYSATS DE LEVURES

### Essai comparatif 1

L'objectif de cet essai est de montrer l'importance du choix de la levure.

Un procédé d'hydrolyse enzymatique identique a été mis en œuvre sur 3 biomasses :
- une biomasse de *Pichia minuta*
- une biomasse de *Pichia heedii*
- une biomasse de *Saccharomyces cerevisiae*

Ce procédé consiste en la mise en œuvre des étapes suivantes :
- solubilisation de la biomasse dans l'eau à raison de 100g/L,
- hydrolyse enzymatique à l'aide d'une protéase,
- inactivation enzymatique par voie thermique à 80°C pendant 1h,
- décantation afin d'éliminer la phase insoluble, récupérer du surnageant,
- filtration, et récupération du filtrat.

Les caractéristiques analytiques des produits obtenus sont présentées dans le tableau ci-dessous :

**Tableau 15. Caractéristiques analytiques des trois hydrolysats de levures**

| | *Hydrolysat de Pichia minuta* | *Hydrolysat de Pichia heedii* | *Hydrolysat de Saccharomyces cerevisiae* |
|---|---|---|---|
| Matières sèches (MS) g/L | 36 | 22 | 24 |
| pH | 5,4 | 5,5 | 5,2 |
| Couleur L,a,b | 99,48 | 99,42 | 99,97 |
| | -0,78 | -1,18 | -0,27 |
| | 3,29 | 3,95 | 0,78 |
| Cendres g/L | 7,8 | 4,6 | 4,1 |
| % cendres / MS | 21.7% | 20.9% | 17.1% |
| Protéines (méthode de Lowry) g/L | 18,6 | 6,8 | 1,5 |
| % Protéines / MS | 52% | 31% | 6% |

On constate que ces hydrolysats ne présentent pas les mêmes caractéristiques analytiques, notamment en ce qui concerne les teneurs en protéines qui sont très différentes pour les 3 produits obtenus à partir du même procédé mais à partir de levures différentes.

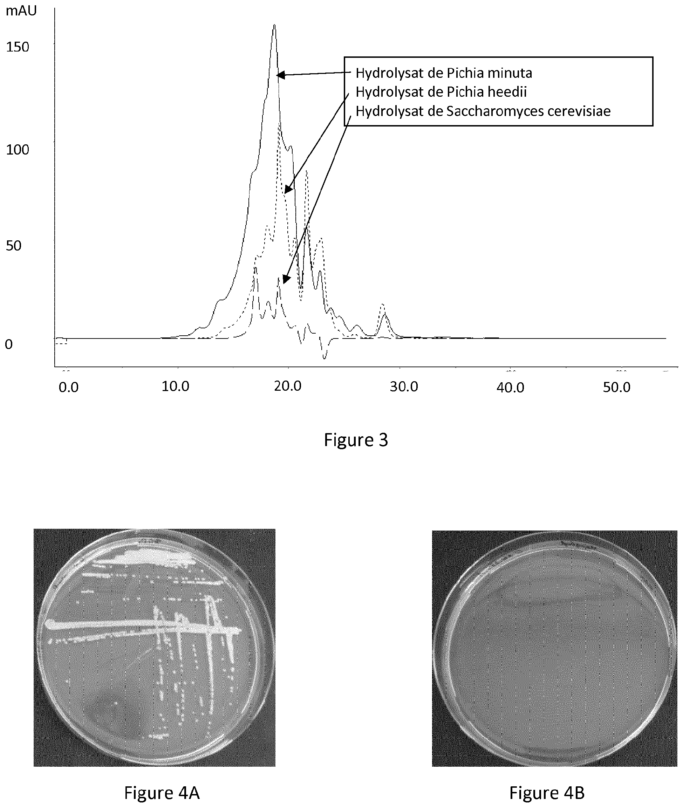

La taille des protéines est également différente, comme on peut le voir sur le chromatogramme des protéines des trois produits, tel que présenté à la Figure 3.

Ces 3 hydrolysats ont ensuite été testés pour leur effet sur l'expression P16, les tests ayant été réalisés selon les conditions et le protocole décrit au point I.2 de la partie évaluation de l'effet cosmétique de la présente demande.

Les résultats obtenus sont présentés dans le Tableau 16.

**Tableau 16. Capacité des trois hydrolysats à inhiber le P16.**

| | **P16 (%)** | **Capacité à limiter l'expression de P16 (%)** |
|---|---|---|
| **Modèle normal** | | |
| Témoin | 100 | |

| **Modèle alopécique** | | |
|---|---|---|
| Témoin | 195 | |
| Hydrolysat de *Pichia minuta* 0,04% | 128 | 71 |
| Hydrolysat de *Pichia heedii* 1% | 222 | 0 |
| Hydrolysat de *Saccharomyces cerevisiae* 1*%* | 214 | 0 |

Ces résultats montrent bien que le choix de la levure est important, puisqu'un même procédé de production de l'actif appliqué à 3 biomasses de levures d'une même famille, aboutit à trois produits différents, dont un seul est efficace sur un paramètre clé dans la lutte contre la chute des cheveux.

### Essai comparatif 2

L'objectif de cet essai est de comparer l'efficacité d'un actif cosmétique, un hydrolysat de *Pichia anomala* décrit dans le brevet FR3016521, à celle du principe actif selon l'invention afin de montrer qu'un ingrédient actif cosmétique issu d'une levure du genre *Pichia,* ne fonctionne pas obligatoirement.

La caractérisation analytique des deux actifs est présentée dans le Tableau 17.

**Tableau 17. Caractéristiques analytiques des deux hydrolysats**

| | *Hydrolysat de Pichia minuta* | *Hydrolysat de Pichia anomala* (FR3016521) |
|---|---|---|
| % Cendres / MS | 26% | 34.7% |
| % Protéines / MS | 59% | 59% |
| % sucres / MS | 15% | 6.3% |

On constate que ces hydrolysats présentent des caractéristiques analytiques similaires, notamment en ce qui concerne les teneurs en protéines qui sont identiques.

La caractérisation de la fraction protéique est présentée dans le Tableau 18.

**Tableau 18. Caractérisation de la fraction protéique des deux hydrolysats**

| | *Hydrolysat de Pichia anomala* (FR3016521) | | *Hydrolysat de Pichia minuta* |
|---|---|---|---|
| Fraction 1 : Mm > 5000Da | 13.0% | Fraction 1 : Mm > 10 000Da | 0.3% |
| Fraction 2 : 2000<M m<5000Da | 38.4% | Fraction 2 : 3500<Mm<10 000Da | 2.9% |
| | | Fraction 3 : 2000<Mm<3500Da | 10.5% |
| Fraction 3 : 243<Mm<2000Da | 46.3% | Fraction 4 : 243<Mm<2000Da | 83.3% |
| Fraction 4 : 0<Mm<243Da | 2.3% | Fraction 5 : 0<Mm<243Da | 3.0% |

La taille des protéines est également similaire, puisque les deux hydrolysats contiennent 59% de protéines, dont plus de 87% présentent un poids moléculaire inférieur à 5kDa.

Ces 2 hydrolysats ont ensuite été testés pour leur effet sur l'expression P16, les tests ayant été réalisés selon les conditions et le protocole décrit au point I.2 de la partie évaluation de l'effet cosmétique de la présente demande.

Les résultats obtenus sont présentés dans le Tableau 19.

**Tableau 19. Capacité des deux hydrolysats à inhiber le P16.**

| | **P16 (%)** | **Capacité à limiter l'expression de P16 (%)** |
|---|---|---|
| **Modèle normal** | | |
| Témoin | 100 | |

| **Modèle alopécique** | | |
|---|---|---|
| Témoin | 195 | |
| Hydrolysat de Pichia minuta 0,04% | 128 | 71 |
| Hydrolysat de Pichia anomala 1% | 225 | 0 |

Ces résultats montrent encore qu'il est évident que le choix de la levure est important, puisque un hydrolysat de *Pichia anomala* issu d'une levure du genre *Pichia,* présentant en outre les mêmes teneurs en protéines que l'hydrolysat de *Pichia minuta* auquel il est comparé, ne présente pas d'efficacité sur le marqueur capillaire choisi.

### Essai comparatif 3

L'objectif de cet essai est de comparer l'efficacité d'un hydrolysat Torulaspora delbrueckii, décrit dans le brevet FR2979541, à celle du principe actif selon l'invention de l'exemple 1, afin de montrer qu'un ingrédient actif cosmétique issu d'une levure présentant une caractéristique analytique similaire, ne fonctionne pas obligatoirement.

La caractérisation analytique des deux actifs est présentée dans le Tableau 20.

**Tableau 20. Caractéristiques analytiques des deux hydrolysats**

| | Hydrolysat de Pichia minuta | Hydrolysat de Torulaspora delbrueckii (FR2979541) |
|---|---|---|
| % cendres / MS | 26% | 28.3% |
| % Protéines / MS | 59% | 64.3% |
| % sucres / MS | 15% | 6.95% |
| % Polyphenols / MS | 0% | 0.25% |

On constate que ces hydrolysats présentent des caractéristiques analytiques similaires, notamment en ce qui concerne les teneurs en protéines qui sont proches.

La caractérisation de la fraction protéique est présentée dans le Tableau 21.

**Tableau 21. Caractérisation de la fraction protéique des deux hydrolysats**

| | Hydrolysat de Torulaspora delbrueckii (FR2979541) | | Hydrolysat de Pichia minuta |
|---|---|---|---|
| Fraction 1 : Mm > 5000Da | 3.3% | Fraction 1 : Mm > 10 000Da | 0.3% |
| Fraction 2 : 2000<Mm≤5000Da | 25.64% | Fraction 2 : 3500<Mm≤10 000Da | 2.9% |
| | | Fraction 3 : 2000<Mm≤3500Da | 10.5% |
| Fraction 3 : 243<Mm≤2000Da | 68.29% | Fraction 4 : 243<Mm≤2000Da | 83.3% |
| Fraction 4 : 0<Mm≤243Da | 2.77% | Fraction 5 : 0<Mm≤243Da | 3.0% |

La taille des protéines est également similaire, puisque les deux hydrolysats contiennent plus de 93% présentent un poids moléculaire inférieur à 5kDa.

Ces 2 hydrolysats ont ensuite été testés pour leur effet sur l'expression P16, les tests ayant été réalisés selon les conditions et le protocole décrit au point I.2 de la partie évaluation de l'effet cosmétique de la présente demande.

Les résultats obtenus sont présentés dans le Tableau 22.

**Tableau 22. Capacité des deux hydrolysats à inhiber le P16.**

| | **P16 (%)** | **Capacité à limiter l'expression de P16 (%)** |
|---|---|---|
| **Modèle normal** | | |
| Témoin | 100 | |

| **Modèle alopécique** | | |
|---|---|---|
| Témoin | 195 | |
| Hydrolysat de Pichia minuta 0,04% | 128 | 71 |
| Hydrolysat de Torulaspora delbrueckii 1% | 201 | 0 |

Ces résultats montrent encore que le choix de la levure est important, puisqu'un hydrolysat d'une autre levure présentant une caractérisation analytique similaire (teneur protéines/cendres/sucres) et des tailles de protéines identiques (93% de protéines < 5000Da), ne présente pas d'efficacité sur le marqueur capillaire choisi.

### Essai comparatif 4

Cet essai a pour objectif de démontrer qu'un hydrolysat de *Pichia minuta,* ne présente pas les mêmes caractéristiques analytiques et est par conséquent différent d'une biomasse de *Pichia minuta,* telle qu'elle existe dans la nature.

Tout d'abord, l'hydrolysat ne contient pas les levures vivantes alors que la biomasse en contient.

Le procédé de culture de microorganismes sur gélose, permet ce contrôle. Il a été mis en œuvre pour l'hydrolysat de l'exemple 1 d'une part et pour une biomasse de *Pichia minuta* d'autre part :
- Isolement par strie en cadran à l'oese de 1µL,
- Milieu de culture : Sabouraud,
- Incubation 48h à 30°C

Les résultats sont présentés sur la Figure 4B pour l'hydrolysat et sur la Figure 4A pour la biomasse.

On ne constate (Figure 4B) pas de croissance sur la gélose avec l'hydrolysat de l'exemple 1, alors qu'en revanche on observe (Figure 4A) une croissance des colonies blanches, rondes, brillantes, grasses, de diamètre inférieur à 0,5mm sur la gélose avec la biomasse de *Pichia minuta.*

La caractérisation analytique de l'hydrolysat de l'exemple 1 et d'une biomasse de *Pichia minuta* est en outre présentée dans le Tableau 23.

**Tableau 23. Caractéristiques analytiques de l'hydrolysat selon l'invention et d'une biomasse de Pichia minuta**

| | Hydrolysat de *Pichia minuta* (Exemple 1) | Biomasse de *Pichia minuta* |
|---|---|---|
| % cendres / MS | 26% | 8% |
| % Protéines / MS | 59% | 41% |
| % sucres / MS | 15% | 36% |
| Non Déterminé | - | 15% |

Ces résultats montrent bien qu'il existe des différences analytiques importantes entre une biomasse et un hydrolysat de *Pichia minuta.* La biomasse constitue la matière première naturelle sur laquelle on applique le procédé d'hydrolyse enzymatique pour obtenir le principe actif selon l'invention, l'hydrolysat de Pichia minuta.

## Revendications

1. Principe actif cosmétique **caractérisé en ce qu'**il s'agit d'un hydrolysat de *Pichia minuta.*

2. Principe actif cosmétique selon la revendication 1, caractérisé que l'hydrolysat de *Pichia minuta* comprend au moins des peptides.

3. Principe actif cosmétique selon la précédente revendication, **caractérisé en ce qu'**au moins 50% en poids des composés peptidiques de l'hydrolysat est constitué par des peptides ayant un poids moléculaire inférieur à 3500 Da.

4. Principe actif cosmétique selon l'une des précédentes revendications, **caractérisé en ce qu'**il comprend des peptides de poids moléculaire inférieur à 3500 Da et **en ce que** ces peptides de poids moléculaire inférieur à 3500 Da représentent au moins 40% en poids de la matière sèche totale de l'hydrolysat.

5. Principe actif cosmétique selon l'une des précédentes revendications, **caractérisé en ce que** l'hydrolysat comprend des sucres et **en ce que** les sucres représentent au moins 5% en poids de matière sèche totale de l'hydrolysat.

6. Principe actif cosmétique selon l'une des précédentes revendications, **caractérisé en ce que** l'hydrolysat comprend également des minéraux.

7. Principe actif cosmétique selon l'une des précédentes revendications, **caractérisé en ce qu'**il se présente sous forme liquide.

8. Principe actif cosmétique selon l'une des précédentes revendications, **caractérisé en ce que** l'hydrolysat est un hydrolysat enzymatique ou chimique.

9. Principe actif cosmétique selon l'une des revendications 1 à 8, **caractérisé en ce qu'**il se présente sous forme solide et **en ce qu'**il est constitué par l'hydrolysat de *Pichia minuta* et par un support d'atomisation choisi parmi la maltodextrine, la gomme arabique, la lécithine de soja ou l'isomalt.

10. Principe actif cosmétique selon la revendication 9, **caractérisé en ce que** l'hydrolysat de *Pichia minuta* représente entre 25 et 75% en poids du principe actif.

11. Utilisation non-thérapeutique d'un principe actif selon l'une des revendication 1 à 10 par application sur les cheveux et / ou le cuir chevelu, pour lutter contre la chute des cheveux et activer leur repousse.

12. Principe actif selon l'une des revendications 1 à 10 destiné à être utilisé dans une méthode permettant de traiter une alopécie androgénétique.

13. Utilisation non-thérapeutique d'un principe actif selon la revendication 11 pour stimuler la croissance du follicule pileux.

14. Composition cosmétique comprenant au moins 0,05% en poids d'un principe actif selon l'une des revendications 1 à 10.

15. Composition cosmétique selon la revendication 14, **caractérisée en ce qu'**elle se présente sous forme de lotion, shampoing, crème ou mousse.

16. Procédé cosmétique non-thérapeutique pour lutter contre la chute des cheveux et activer leur repousse, **caractérisé en ce qu'**il consiste à appliquer sur les cheveux ou le cuir chevelu une composition selon l'une des revendications 14 ou 15.

17. Procédé cosmétique non-thérapeutique pour lutter contre la chute des cheveux et activer leur repousse, **caractérisé en ce qu'**il consiste à appliquer sur les cheveux ou le cuir chevelu au moins une fois par jour pendant au moins un mois une composition selon l'une des revendications 14 ou 15

18. Composition selon l'une des revendications 14 ou 15 destinée à être utilisée dans une méthode permettant de traiter une alopécie androgénétique.

## Patentansprüche

1. Kosmetischer Wirkstoff, **dadurch gekennzeichnet, dass** es sich um ein *Pichia minuta-*Hydrolysat handelt.

2. Kosmetischer Wirkstoff nach Anspruch 1, **dadurch gekennzeichnet, dass** das *Pichia minuta*-Hydrolysat mindestens Peptide umfasst.

3. Kosmetischer Wirkstoff nach dem vorhergehenden Anspruch, **dadurch gekennzeichnet, dass** mindestens 50 Gew.-% der Peptidverbindungen des Hydrolysats aus Peptiden mit einem Molekulargewicht von weniger als 3500 Da bestehen.

4. Kosmetischer Wirkstoff nach einem der vorhergehenden Ansprüche, **dadurch gekennzeichnet, dass** er Peptide mit einem Molekulargewicht von weniger als 3500 Da umfasst und dass diese Peptide mit einem Molekulargewicht von weniger als 3500 Da mindestens 40 Gew.-% der Gesamttrockensubstanz des Hydrolysats ausmachen.

5. Kosmetischer Wirkstoff nach einem der vorhergehenden Ansprüche, **dadurch gekennzeichnet, dass** das Hydrolysat Zucker umfasst und der Zucker mindestens 5 Gew.-% der Gesamttrockensubstanz des Hydrolysats ausmacht.

6. Kosmetischer Wirkstoff nach einem der vorhergehenden Ansprüche, **dadurch gekennzeichnet, dass** das Hydrolysat auch Mineralien umfasst.

7. Kosmetischer Wirkstoff nach einem der vorhergehenden Ansprüche, **dadurch gekennzeichnet, dass** er in flüssiger Form vorliegt.

8. Kosmetischer Wirkstoff nach einem der vorhergehenden Ansprüche, **dadurch gekennzeichnet, dass** das Hydrolysat ein enzymatisches oder chemisches Hydrolysat ist.

9. Kosmetischer Wirkstoff nach einem der Ansprüche 1 bis 8, **dadurch gekennzeichnet, dass** er in fester Form vorliegt und dass er aus *Pichia minuta*-Hydrolysat und einem Zerstäubungsträger, ausgewählt aus Maltodextrin, Gummi arabicum, Sojalecithin oder Isomalt, besteht.

10. Kosmetischer Wirkstoff nach Anspruch 9, **dadurch gekennzeichnet, dass** das *Pichia minuta*-Hydrolysat zwischen 25 und 75 Gew.-% des Wirkstoffs ausmacht.

11. Nicht-therapeutische Verwendung eines Wirkstoffs nach einem der Ansprüche 1 bis 10 durch Auftragen auf das Haar und/oder die Kopfhaut zur Bekämpfung von Haarausfall und zur Aktivierung des Haarnachwuchses.

12. Wirkstoff nach einem der Ansprüche 1 bis 10 zur Verwendung in einem Verfahren zur Behandlung von androgenetischer Alopezie.

13. Nicht-therapeutische Verwendung eines Wirkstoffs nach Anspruch 11 zur Stimulierung des Wachstums des Haarfollikels.

14. Kosmetische Zusammensetzung, umfassend mindestens 0,05 Gew.-% eines Wirkstoffs nach einem der Ansprüche 1 bis 10.

15. Kosmetische Zusammensetzung nach Anspruch 14, **dadurch gekennzeichnet, dass** sie in Form einer Lotion, eines Shampoos, einer Creme oder eines Schaums vorliegt.

16. Nicht-therapeutisches kosmetisches Verfahren zur Bekämpfung von Haarausfall und zur Aktivierung des Haarnachwuchses, **dadurch gekennzeichnet, dass** es im Auftragen einer Zusammensetzung nach einem der Ansprüche 14 oder 15 auf das Haar oder die Kopfhaut besteht.

17. Nicht-therapeutisches kosmetisches Verfahren zur Bekämpfung von Haarausfall und zur Aktivierung des Haarnachwuchses, **dadurch gekennzeichnet, dass** es im Auftragen einer Zusammensetzung nach einem der Ansprüche 14 oder 15 auf das Haar oder die Kopfhaut mindestens einmal täglich für mindestens einen Monat besteht.

18. Zusammensetzung nach einem der Ansprüche 14 oder 15 zur Verwendung in einem Verfahren zur Behandlung von androgenetischer Alopezie.

## Claims

1. Cosmetic active ingredient, **characterized in that** it is a hydrolyzate of *Pichia minuta.*

2. Cosmetic active ingredient according to claim 1, **characterized in that** the hydrolyzate of *Pichia minuta* comprises at least peptides.

3. Cosmetic active ingredient according to the preceding claim, **characterized in that** at least 50% by weight of the peptide compounds of the hydrolyzate is composed of peptides having a molecular weight of less than 3500 Da.

4. Cosmetic active ingredient according to any of the preceding claims, **characterized in that** it comprises peptides having a molecular weight of less than 3500 Da and **in that** these peptides having a molecular weight of less than 3500 Da represent at least 40% by weight of the total dry matter of the hydrolyzate.

5. Cosmetic active ingredient according to any of the preceding claims, **characterized in that** the hydrolyzate comprises sugars and **in that** the sugars represent at least 5 wt.% of the total dry matter of the hydrolyzate.

6. Cosmetic active ingredient according to any of the preceding claims, **characterized in that** the hydrolyzate also comprises minerals.

7. Cosmetic active ingredient according to any of the preceding claims, **characterized in that** it is in liquid form.

8. Cosmetic active ingredient according to any of the preceding claims, **characterized in that** the hydrolyzate is an enzymatic or chemical hydrolyzate.

9. Cosmetic active ingredient according to any of claims 1 to 8, **characterized in that** it is in solid form and **in that** it is composed of the hydrolyzate of *Pichia minuta* and an atomization support selected from maltodextrin, gum arabic, soy lecithin or isomalt.

10. Cosmetic active ingredient according to claim 9, **characterized in that** the hydrolyzate of *Pichia minuta* represents between 25 and 75% by weight of the active ingredient.

11. Non-therapeutic use of an active ingredient according to any of claims 1 to 10 by application to the hair and/or the scalp, for combatting hair loss and activating hair regrowth.

12. Active ingredient according to any of claims 1 to 10, intended for use in a method for treating androgenetic alopecia.

13. Non-therapeutic use of an active ingredient according to claim 11 for stimulating the growth of the hair follicle.

14. Cosmetic composition comprising at least 0.05% by weight of an active ingredient according to any of claims 1 to 10.

15. Cosmetic composition according to claim 14, **characterized in that** it is in the form of a lotion, shampoo, cream or mousse.

16. Non-therapeutic cosmetic method for combatting hair loss and activating hair regrowth, **characterized in that** it involves applying a composition according to either claim 14 or claim 15 to the hair or the scalp.

17. Non-therapeutic cosmetic method for combatting hair loss and activating hair regrowth, **characterized in that** it involves applying a composition according to either claim 14 or claim 15 to the hair or the scalp at least once a day for at least one month.

18. Composition according to either claim 14 or claim 15, intended for use in a method for treating androgenetic alopecia.
